# EUROPÄISCHE PATENTSCHRIFT

(11) **EP 0 699 056 B1**
(45) Veröffentlichungstag und Bekanntmachung des Hinweises auf die Patenterteilung: **29.01.1997**
(21) Anmeldenummer: 94918336.2
(22) Anmeldetag: 18.05.1994
(51) Int. Cl.: A61B 17/60

(54) **KNOCHENCHIRURGISCHE HALTEVORRICHTUNG**
HOLDING DEVICE FOR USE IN BONE SURGERY
DISPOSITIF DE FIXATION UTILISE EN CHIRURGIE OSSEUSE

(30) Priorität: 18.05.1993 DE 4316541; 22.09.1993 DE 9314294 U
(43) Veröffentlichungstag der Anmeldung: 06.03.1996
(73) Patentinhaber: Schäfer micomed GmbH, D-73614 Schorndorf (DE)
(72) Erfinder: STÜCKER, Ralf, D-79183 Waldkirch (DE); REHDER, Günther, D-73650 Winterbach (DE)
(74) Vertreter: Steimle, Josef, Dipl.-Ing.
(86) Internationale Anmeldenummer: EP9401606
(87) Internationale Veröffentlichungsnummer: WO9426194

(56) Entgegenhaltungen:
- EP-A- 0 443 894
- US-A- 5 176 678

## Beschreibung

Die Erfindung betrifft eine knochenchirurgische Haltevorrichtung für einen Korrekturstab, mit einer am Knochen befestigbaren Knochenplatte, die ein Lager für eine Aufnahmevorrichtung für den Korrekturstab aufweist, und mit einer den Korrekturstab in der Aufnahmevorrichtung haltenden Fixiereinrichtung.

Knochenchirurgische Haltevorrichtungen sind in einer Vielzahl bekannt. Zur Fixierung einzelner Knochen bei Deformationen oder Frakturen werden in der Regel Knochenschrauben verwendet, die in die Knochen bzw. Knochensegmente eingeschraubt und über Korrekturstäbe miteinander verbunden werden. Diese Knochenschrauben bestehen in der Regel aus einem Gewindeschaft und einem Schraubenkopf, wobei der Gewindeschaft bis zur Anlage des Schraubenkopfes auf der Knochenoberfläche in den Knochen eingeschraubt wird. Diese-Lage der Knochenschraube ist anzustreben, da in dieser Stellung ein Maximum an Zug- und Druckkräften sowie Momenten auf den Knochen übertragen werden kann. Da der Schraubenkopf der Knochenschraube in der Regel als Gabelkopf ausgebildet ist, muß die Knochenschraube eine Drehstellung einnehmen, in der die Nut zwischen den Schenkeln des Gabelkopfes in Richtung des aufzunehmenden Korrekturstabes liegt. Aufgrund dieser Anforderung ist jedoch nicht immer gewährleistet, daß die Knochenschraube vollständig bis zur Anlage des Schraubenkopfes an der Knochenoberfläche eingeschraubt ist. Abhängig von der Gewindesteigung befindet sich der Schraubenkopf in einem mehr oder weniger großen Abstand von der Knochenoberfläche, wodurch einerseits die Höhe der aufzubringenden Zug- und Druckkräfte eingeschränkt wird, andererseits die Gefahr einer Schädigung des Knochens bei Kraftspitzen vergrößert wird.

Befindet sich zwischen dem Schraubenkopf und dem Knochen eine Knochenplatte und wird diese Knochenplatte über die Knochenschrauben mit fixiert, dann ist nicht immer gewährleistet, daß die Knochenplatte, die die Funktion einer gleichmäßigen Kräfteverteilung der an der Knochenschraube wirkenden Kräfte besitzt, über die Knochenschraube spielfrei und satt anliegend auf die Oberfläche des Knochens gedrückt wird. Liegt die Knochenplatte mit Spiel auf dem Knochen auf, dann besteht sogar die Gefahr von Knochenreizungen, was den Heilungs- prozeß verlangsamt. Die Knochenplatte wird vom Schrauben- kopf der Knochenschraube nur dann spielfrei am Knochen fixiert, wenn zufällig die gewünschte Drehstellung der Knochenschraube, die abhängt von der Ausrichtung des Korrekturstabes, dann eingenommen wird, wenn die maximale Einschraubtiefe erreicht ist.

Mit der EP-A-443 894 ist eine Knochenplatte bekannt geworden, die über zwei Knochenschrauben am Knochen fixierbar ist. Das Lager für den Fixierstab weist eine Richtung auf, die von der Lage der Knochenplatte am Knochen abhängig ist.

Der Erfindung liegt daher die Aufgabe zugrunde, eine Haltevorrichtung der eingangs genannten Art so weiterzubilden, daß mit ihr die Knochenplatte unabhängig von der Drehstellung der Aufnahmevorrichtung für den Korrekturstab spielfrei am Knochen anliegt.

Diese Aufgabe wird erfindungsgemäß dadurch gelöst, daS die Aufnahmevorrichtung über die proximale Seite in das Lager einschiebbar ist und dabei die Knochenplatte durchgreift und auf der distalen Seite zur Aufnahme des Korrekturstabes überragt und die Knochenplatte mit eingeschobener Aufnahmevorrichtung unabhängig von der Drehstellung der Aufnahmevorrichtung am Knochen fixierbar ist.

Durch die erfindungsgemäße Ausgestaltung der Haltevorrichtung, bei der die Knochenplatte unabhängig von der Aufnahmevorrichtung am Knochen fixierbar ist, was z.B. über zwei oder mehrere Schrauben erfolgt, wird der Vorteil erzielt, daß die Korrekturkräfte bzw. Momente spielfrei von der Knochenplatte auf einen großen Bereich des Knochens übertragen werden. Da die Fixierung der Knochenplatte unabhängig von der Aufnahmevorrichtung ist, spielt die Lage bzw. die Ausrichtung des Korrekturstabes keine Rolle.

Die Aufnahmevorrichtung ist mit der Knochenplatte durch Einschub von der proximalen Seite, d.h. von der Seite, die an der Oberfläche des Knochens anliegt, verbunden, wobei sie von einem Lager der Knochenplatte gehalten wird. Die Aufnahmevorrichtung überragt die Knochenplatte an der distalen Seite und kann dort den Korrekturstab aufnehmen. Vorteilhaft ist die Aufnahmevorrichtung in mehreren Stufen oder stufenlos drehbar im Lager angeordnet. Dadurch kann die Aufnahmevorrichtung exakt in Richtung des Korrekturstabes ausgerichtet werden, wobei die Drehstellung unabhängig von der Fixierung der Knochenplatte ist.

Gemäß einem Ausführungsbeispiel vorliegender Erfindung weist die Knochenplatte eine kreisrunde Durchgangsöffnung auf, in der die Aufnahmevorrichtung radial unbeweglich gehalten ist. Demgegenüber ist gemäß einem weiteren bevorzugten Ausführungsbeispiel vorliegender Erfindung die Aufnahmevorrichtung im Lager in einer Richtung quer verschiebbar. Auf diese Weise müssen drei oder mehr Knochenplatten, deren Aufnahmevorrichtungen durch einen einzigen Korrekturstab miteinander verbunden werden sollen, nicht unbedingt in genauer Flucht zueinander gebracht werden, was ohnehin schwierig ist. Es ist auf diese Weise stattdessen möglich, die Aufnahmevorrichtungen nicht nur zu verdrehen, sondern auch in einer Achsrichtung zu verschieben, um eine genaue Flucht von drei oder mehr solcher Aufnahmevorrichtungen zu erreichen. Dies läßt ein weniger aufwendiges Arbeiten beim Ausrichten und Fixieren der Knochenplatten am Knochen zu.

Bei der letztgenannten weiteren bevorzugten Ausführungsform ist es konstruktiv zweckmäßig, wenn die Knochenplatte eine langlochartige Durchgangsöffnung aufweist, in der die Aufnahmevorrichtung quer beweglich gehalten ist, und daß das Lager entsprechend der Durchgangsöffnung ebenfalls langlochartig ausgebildet ist.

Bevorzugt schließt die Aufnahmevorrichtung auf der proximalen Seite der Knochenplatte bündig mit dieser ab oder ist versenkt angeordnet. Hierdurch wird gewährleistet, daß eine Verdrehung der Aufnahmevorrichtung zur Ausrichtung bei angeschraubter Knochenplatte nicht zu Verletzungen der Knochenoberfläche führt. Außerdem wirkt sich die Aufnahmevorrichtung durch den bündigen Abschluß bzw. durch die versenkte Anordnung beim Anschrauben der Knochenplatte nicht störend aus.

Bei einer bevorzugten Weiterbildung ist vorgesehen, daß die Knochenplatte mehrere Lager für entsprechend mehrere Aufnahmevorrichtungen aufweist. Dies ist insbesondere bei größeren Knochenplatten der Fall, wo ein Korrekturstab an mehreren Stellen fixiert werden muß bzw. mehrere Korrekturstäbe an der Knochenplatte angeordnet werden. Auch hier können die einzelnen Aufnahmevorrichtungen individuell auf den jeweiligen Korrekturstab ausgerichtet werden, ohne daß die Knochenplatte hiervon beeinflußt wird.

Vorzugsweise ist die Aufnahmevorrichtung als Gabelkopf ausgebildet und weist an ihrem in der Knochenplatte zu verankernden Ende einen radial vorspringenden Bund auf, der das Lager der Knochenplatte hintergreift. Der das Lager hintergreifende Bund verhindert das Herausziehen der Aufnahmevorrichtung durch die Knochenplatte und stellt gleichzeitig das Drehlager bei drehbaren Aufnahmevorrichtungen dar. Dabei kann der Bund kreisringförmig oder vieleckig sein, wobei, wie bereits erwähnt, bei der Kreisringform eine stufenlose Verdrehung möglich ist, bei einer fünfeckigen bzw. siebeneckigen Form entsprechend viele Drehstellungen möglich sind.

In Weiterbildung der Erfindung ist ein Überwurfring vorgesehen, der die Aufnahmevorrichtung aufnimmt. Dabei ist beim vorgenannten ersten Ausführungsbeispiel vorliegender Erfindung der Überwurfring auf der distalen Seite der Knochenplatte aufliegend angeordnet und die an der Knochenplatte anliegende Fläche des Überwurfringes und die distale Seite der Knochenplatte sind mit einer das Lager umgebenden radialen Stirnverzahnung (Hirth-Verzahnung) versehen. Gemäß dem vorgenannten zweiten Ausführungsbeispiel vorliegender Erfindung ist dagegen ein Zwischenring vorgesehen, der die Aufnahmevorrichtung aufnimmt, wobei zweckmäßigerweise der Zwischenring zwischen der distalen Seite der Knochenplatte und der proximalen Seite des Überwurfringes angeordnet ist. Hierbei sind aneinander liegende Stirnseiten von Überwurfring und Zwischenring mit einer radialen Stirnverzahnung (Hirth-Verzahnung) versehen, während die an der Knochenplatte anliegende Fläche des Zwischenringes und die distale Seite der Knochenplatte eine das langlochartige Lager umgebende Stirnverzahnung aufweisen, die jeweils durch quer zur Längserstreckung des Lagers verlaufende parallele Kerben gebildet ist. Über die radiale Stirnverzahnung wird somit eine Verdrehsicherung des Überwurfringes bezüglich der Knochenplatte entweder unmittelbar oder mittelbar über den Zwischenring geschaffen. Die parallelen Kerben schaffen demgegenüber beim zweiten Ausführungsbeispiel vorliegender Erfindung eine Sicherung der verschobenen Position der Aufnahmevorrichtung bezüglich der Knochenplatte über den Zwischenring.

Bei der vorgenannten zweiten Ausführungsform vorliegender Erfindung ist außerdem zwischen der Aufnahmevorrichtung und dem Überwurfring eine zwar drehfeste jedoch axial bewegliche Verbindung vorgesehen, um zu gewährleisten, daß durch das mittelbare Festlegen des Überwurfringes auch der Gabelkopf der Aufnahmevorrichtung festgelegt ist. Dies erfolgt in einfacher Weise dadurch, daß die Aufnahmevorrichtung an mindestens einer Umfangsstelle eine axiale Nut aufweist, in die ein eine Radialbohrung des Überwurfringes durchdringender Stift oder dgl. umfangsseitig unbeweglich eingreift.

Ferner weist die distale Seite des Überwurfringes eine Diagonalnut zur Aufnahme des Korrekturstabes auf. Bei in die Aufnahmevorrichtung eingelegtem Korrekturstab liegt dieser in der Diagonalnut des Überwurfringes und ist zusätzlich von den beiden Schenkeln des Gabelkopfes der Aufnahmevorrichtung umgeben. Auf diese Weise wird über die Stirnverzahnung und die Diagonalnut die Richtung des Überwurfringes und somit des Gabelkopfes festgelegt.

Vorteilhaft ist die Diagonalnut insbesondere mit 0hinterschneidungsfreien Längskerben versehen, in die der mit zu den Längskerben entsprechenden Längsnuten versehene Korrekturstab einsetzbar ist. Durch die Längskerben und Längsnuten des Überwurfringes bzw. des Korrekturstabes wird zusätzlich eine Verdrehsicherung des Korrekturstabes um seine Längsachse geschaffen.

Zur Befestigung des Korrekturstabes in der Aufnahmevorrichtung weist die Fixiereinrichtung eine auf die als Gabelkopf ausgebildete Aufnahmevorrichtung aufschraubbare Kopfmutter, insbesondere eine Hutmutter auf. Durch diese aufschraubbare Kopfmutter wird der Korrekturstab derart in der Aufnahmevorrichtung fixiert, daß die auftretenden Zug- und Druckkräfte sowie die Momente an die Aufnahmevorrichtung und von dieser an die Knochenplatte übertragen werden können.

Bevorzugt ist die Kopfmutter mit einer koaxial eingeschraubten Fixierschraube, insbesondere mit einem Innensechskant und/oder einer Ringschneide versehen. Über den Innensechskant kann nach dem Aufschrauben der Kopfmutter die Fixierschraube verstellt und derart auf den eingelegten Fixierstab aufgeschraubt werden, daß die Ringschneide in das Material des Fixierstabes eingräbt. Auf diese Weise wird eine Fixierung sowohl gegen Verschieben als auch gegen Verdrehen geschaffen.

Bei einer Weiterbildung ist vorgesehen, daß die Knochenplatte wenigstens zwei bezüglich des Lagers einander gegenüberliegende Befestigungsbohrungen zur Aufnahme von Knochenschrauben aufweist. Über diese Knochenschrauben wird die Knochenplatte am Knochen fixiert, wobei die Befestigungsbohrungen zur Aufnahme des Schraubenkopfes der Knochenschrauben dienen.

Vorteilhaft ist dabei die proximale Einsenkung orthogonal zur Oberfläche der Knochenplatte und die distale Befestigungsbohrung schräg angeordnet, so daß die Knochenschrauben konvergieren. Dies hat den Vorteil, daß die Knochenplatte auch bei relativ kleinen Knochen noch sicher befestigbar ist und die Schrauben nicht unmittelbar nach dem Einschrauben wieder auf der anderen Seite des Knochens austreten.

Bei einer besonders bevorzugten Weiterbildung weist die Befestigungsbohrung ein kalottenförmiges Lager für den Schraubenkopf der Knochenschraube auf. Hierdurch wird die Möglichkeit geschaffen, daß die Knochenschraube nicht ausschließlich orthogonal zur Knochenplatte eingeschraubt werden muß, sondern geringfügige Schrägstellungen einnehmen kann, so daß der Schraubenschaft unabhängig von der Lage der Knochenplatte in Bereiche des Knochens eingedreht werden kann, in denen er einen optimalen Halt findet kann.

Weitere Vorteile, Merkmale und Einzelheiten der Erfindung ergeben sich aus der nachfolgenden Beschreibung, in der unter Bezugnahme auf die Zeichnung zwei bevorzugte Ausführungsbeispiele im einzelnen dargestellt sind. In der Zeichnung zeigen:
- Figur 1: eine Draufsicht auf eine Knochenplatte der Haltevorrichtung nach Fig. 6;
- Figur 2: eine Seitenansicht eines Gabelkopfes der Haltevorrichtung nach Fig. 6;
- Figur 3: eine Seitenansicht eines Überwurfringes der Haltevorrichtung nach Fig. 6;
- Figur 4: eine Draufsicht auf den Überwurfring gemäß Figur 3;
- Figur 5: eine Draufsicht auf eine Kopfmutter der Haltevorrichtung nach Fig. 6;
- Figur 6: eine teilweise geschnittene Seitenansicht einer knochenchirurgischen Haltevorrichtung mit eingelegtem Korrekturstab gemäß einem ersten Ausführungsbeispiel vorliegender Erfindung;
- Figur 7: eine Draufsicht auf die Haltevorrichtung gemäß Figur 6;
- Figur 8: eine teilweise geschnittene Seitenansicht einer knochenchirurgischen Haltevorrichtung ohne eingelegten Korrekturstab und ohne aufgeschraubter Kopfmutter gemäß einem zweiten Ausführungsbeispiel vorliegender Erfindung;
- Figur 9: eine Draufsicht auf die Knochenplatte der Haltevorrichtung nach Figur 8;
- Figur 10: eine teilweise aufgebrochene Seitenansicht des Gabelkopfes der Haltevorrichtung nach Figur 8;
- Figur 11: eine Unteransicht, einen Längsschnitt bzw. eine Draufsicht auf einen Zwischenring der Haltevorrichtung nach Figur 8;
- Figur 12: eine Unteransicht, einen Längsschnitt bzw. eine Draufsicht auf den Überwurfring der Haltevorrichtung nach Figur 8.

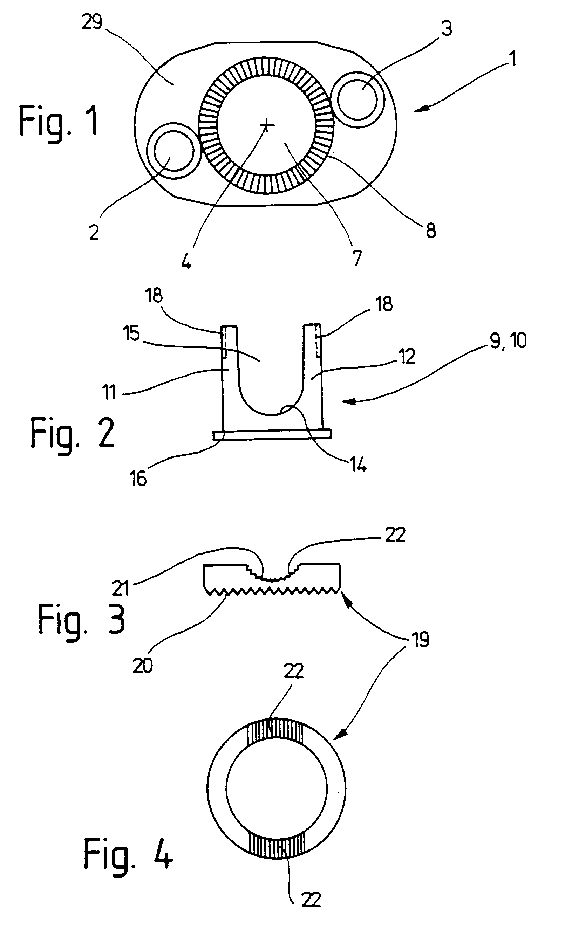

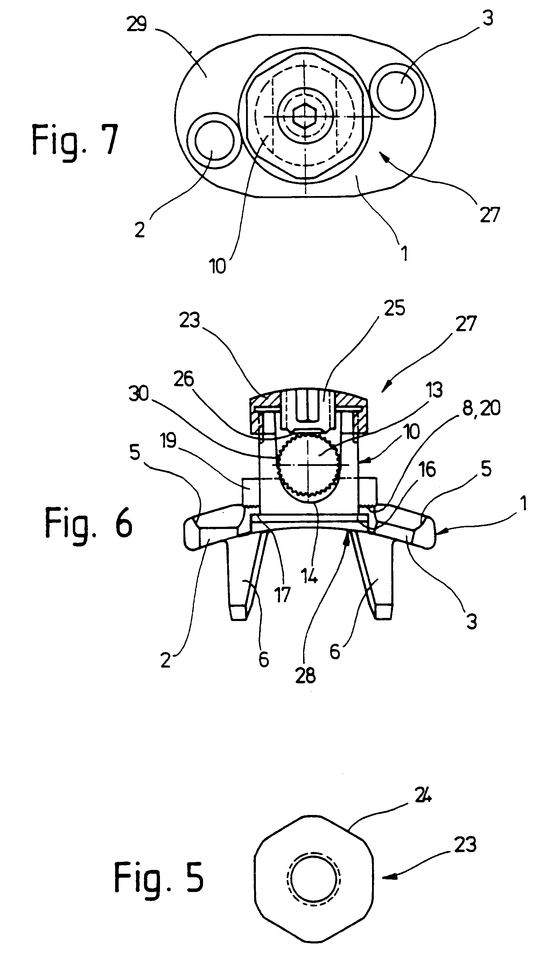

Die Figur 1 zeigt eine Knochenplatte 1 einer knochenchirurgischen Haltevorrichtung (Fig. 6) gemäß dem ersten Ausführungsbeispiel vorliegender Erfindung in Draufsicht, wobei die beiden Befestigungsbohrungen 2 und 3 erkennbar sind, über die die Knochenplatte 1 am Knochen über geeignete Knochenschrauben befestigbar ist. Bevorzugt sind die beiden Befestigungsbohrungen 2 und 3 bezüglich des Zentrums 4 der Knochenplatte 1 einander gegenüberliegend angeordnet. Die Befestigungsbohrungen 2 und 3 weisen jeweils ein kalottenförmiges Lager 5 auf, was deutlich aus Figur 6 erkennbar ist. Von diesem kalottenförmigen Lager 5 wird der Schraubenkopf einer nicht dargestellten Knochenschraube aufgenommen, der entsprechend sphärisch ausgebildet ist. Das Lager 5 hat den Vorteil, daß die Knochenschraube nicht zwingend koaxial zur Befestigungsbohrung 2 bzw. 3 ausgerichtet sein muß, sondern geringe Schrägstellungen einnehmen kann, so daß die Knochenschraube in geeignete Bereiche des Knochens eingeschraubt werden kann.

Ferner ist in Figur 6 erkennbar, daß die Befestigungsbohrung 3 gegenüber der Befestigungsbohrung 2 schräggestellt ist, wobei die Achse der Befestigungsbohrung 3 orthogonal zur Knochenplatte 1 und die Achse der Befestigungsbohrung 2 parallel zu den Ankerstiften 6 verläuft.

In Figur 1 ist außerdem erkennbar, daß die Knochenplatte 1 mit einem zentralen Durchbruch 7 versehen ist, der bei der in der Figur 1 dargestellten Ausführungsform kreisförmig ist. Dieser Durchbruch 7 ist von einer radialen Stirnverzahnung 8 umgeben, die in der distalen Oberseite der Knochenplatte 1 vorgesehen ist.

In Figur 2 ist eine als Gabelkopf 9 ausgebildete Aufnahmevorrichtung 10 dargestellt, die eine im wesentlichen U-förmige Gestalt aufweist. Dabei bilden zwei Schenkel 11 und 12 eine Aufnahme für einen Korrekturstab 13 (Figur 6), der nahe dem Grund 14, der zwischen den Schenkein 11 und 12 gebildeten Nut 15 zu liegen kommt. Der obere Abschnitt eines jeden Schenkels 11 und 12 ist mit einem Außengewinde 18 versehen. An der Unterseite weist der Gabelkopf 9 einen radial vorspringenden Bund 16 auf, der ein Lager 17 an der Unterseite der Knochenplatte 1 hintergreift (Figur 6).

Die Figur 3 zeigt einen Überwurfring 19, der an seiner Unterseite mit einer zur Stirnverzahnung 8 korrespondierenden radialen Stirnverzahnung 20 versehen ist. Die Oberseite weist eine Diagonalnut 21 auf, die mit hinterschneidungsfreien Längskerben 22 (Figur 4) versehen ist.

Die Figur 5 zeigt eine Draufsicht auf eine in Figur 6 im Querschnitt dargestellte Kopfmutter 23, die auf das Außengewinde 18 des Gabelkopfes 9 aufgeschraubt wird. Die Kopfmutter 23 weist einen Außensechskant 24 auf und ist an ihrer Oberseite ballig ausgeführt. In die Kopfmutter 23 ist koaxial eine Fixierschraube 25 (Figur 6) eingeschraubt. Diese Fixierschraube 25 ist als Innensechskantschraube mit balliger Oberseite ausgeführt und weist an der auf dem Korrekturstab 13 aufliegenden Seite eine Ringschneide 26 auf. Die Fixierschraube 25 und die Kopfmutter 23 bilden die Fixiereinrichtung 27 für den Korrekturstab 13.

Die Draufsicht auf die erfindungsgemäße Haltevorrichtung gemäß Figur 7 zeigt die gedrängte und platzsparende Anordnung der einzelnen Elemente auf der Knochenplatte 1, wobei hervorzuheben ist, daß eine Symmetrie eingehalten wird. Die symmetrische Anordnung ist nicht zwingend erforderlich, jedoch anzustreben.

Nachfolgend wird die Verwendung der einzelnen Elemente kurz erläutert. Vor dem Festschrauben der Knochenplatte 1 auf dem zu behandelnden Knochen wird der Gabelkopf 9 von der proximalen Seite 28 soweit eingeschoben, bis der Bund 16 am Lager 17 anliegt. Dann wird die Knochenplatte 1 mittels geeigneter Knochenschrauben am Knochen befestigt.

Der Gabelkopf 9 wird durch Verdrehen um seine Achse so ausgerichtet, daß der Korrekturstab 13 in die Nut 15 zwischen die beiden Schenkel 11 und 12 eingelegt werden kann. Vor dem Einlegen des Korrekturstabes 13 wird der Überwurfring 19 derart auf den Gabelkopf 9 aufgeschoben, daß die Diagonalnut 21 in Richtung der Nut 15 fluchtet. Dabei greift die Stirnverzahnung 20 des Überwurfringes 19 in die Stirnverzahnung 8 der distalen Seite 29 in der Knochenplatte 1. Nach dem Aufschieben und Ausrichten des Überwurfringes 19 wird der Korrekturstab 13 in die Aufnahmevorrichtung 10 eingelegt, wobei sowohl die Drehstellung des Gabelkopfes 9 als auch die Drehlage des Überwurfringes 19 noch korrigiert werden kann. Mit der Kopfmutter 23, die auf das Außengewinde 18 aufgeschraubt wird, wird die Aufnahmevorrichtung 10 nach oben verschlossen. Eine Fixierung des Korrekturstabes 13 erfolgt über die Fixierschraube 25, die in die Kopfmutter 23 eingeschraubt wird. Dabei gräbt sich die Ringschneide 26 in die Längsnuten 30, die zu den Längskerben 22 korrespondierend ausgebildet sind, ein.

Aus Figur 6 ist deutlich erkennbar, daß der Korrekturstab 13 nicht am Grund 14 der Nut 13, sondern auf den Längskerben 22 der Diagonalnut 21 des Überwurfringes 19 aufliegt. Durch das Festziehen der Fixiereinrichtung 27 wird einerseits der Gabelkopf 21 in das Lager 17 gezogen, andererseits wird der Korrekturstab 13 in die Diagonalnut 21 und dadurch der Überwurfring 19 in die Stirnverzahnung 8 der distalen Seite 29 der Knochenplatte 1 gedrängt. Die Aufnahmevorrichtung 10 ist an der Knochenplatte 1 fixiert. Außerdem ist der Korrekturstab 13 sowohl gegen Verschieben als auch gegen Verdrehen um die Stabachse durch die Längskerben 22 und die Längsnuten 30 gesichert. Schließlich ist der Korrekturstab 13 gegen eine Verdrehung um die Achse des Zentrums 4 gesichert, was durch die Stirnverzahnungen 8 und 20 erfolgt.

Von Vorteil ist bei der erfindungsgemäßen Haltevorrichtung die individuelle Einstellung bzw. Ausrichtung der Aufnahmevorrichtung 10, wobei sichergestellt ist, daß die Zug- und Druckkräfte sowie die Momente des Korrekturstabes 13 direkt auf die Knochenplatte 1 und von dieser auf den Knochen übertragen werden.

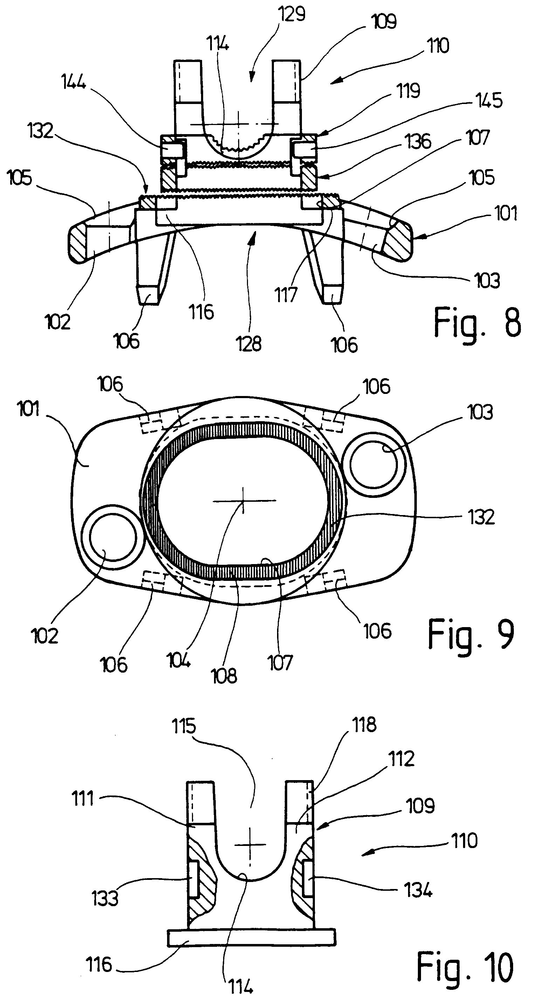

Zur Beschreibung des in Figur 8 dargestellten zweiten Ausführungsbeispieles der knochenchirurgischen Haltevorrichtung werden für ähnliche Teile bzw. Elemente entsprechende Bezugszahlen, denen die Ziffer "1" vorangestellt ist, verwendet. In Figur 8 sind jedoch der Einfachheit halber der einlegbare Korrekturstab 13 und die Fixiereinrichtung 27 weggelassen. Die in Figur 8 dargestellte Haltevorrichtung besitzt außer einer Knochenplatte 101, einer Aufnahmevorrichtung 110, einem Überwurfring 119 und der nicht dargestellten Fixiereinrichtung zwischen dem Überwurfring 119 und der hier mit vier Ankerstiften 106 versehenen Knochenplatte 101 einen auf den Gabelkopf 109 der Aufnahmevorrichtung 110 aufgefädelten Zwischenring 136.

Die Knochenplatte 101 besitzt eine der in Figur 1 dargestellten Knochenplatte 1 entsprechende Grundform mit zwei Befestigungsbohrungen 102 und 103, ist jedoch mit einem als Langloch ausgebildeten Durchbruch 107 versehen, dessen größere Weite sich in der Längserstreckung der Knochenplatte 101 befindet. Um das Langloch 107 ist von der distalen Seite 129 der Knochenplatte 1 her eine mit parallelen Kerben 108 verzahnte Stirn 132 vorgesehen. Die parallelen Kerben 108 verlaufen in Richtung der Schmalseite der Knochenplatte 101, also quer zur Längserstreckung der Knochenplatte 101 bzw. des Langlochs 107.

Die als Gabelkopf 109 ausgebildete Aufnahmevorrichtung 110 entspricht in ihrem wesentlichen Aufbau der in Figur 2 dargestellten Aufnahmevorrichtung 10. Der einzige Unterschied besteht darin, daß sie mit zwei diagonal gegenüberliegenden axial verlaufenden Nuten 133, 134 versehen ist. Der Gabelkopf 109 ist ebenso wie sein Durchmesser größerer Bund 116 im Querschnitt kreisförmig derart, daß die Aufnahmevorrichtung 110 in das Langloch 108 der Knochenplatte 101 von der proximalen Seite 128 her einsteckbar ist, wobei der Bund 116 in einem der Form des Langlochs 107 entsprechenden ovalen Lager 117 aufgenommen ist. Auf diese Weise kann die Aufnahmevorrichtung 110 im Langloch-Durchbruch 107 der Knochenplatte 101 in Richtung der Längserstreckung der Knochenplatte 101 bewegt werden. In Querrichtung der Knochenplatte 101 ist die Aufnahmevorrichtung 110 fixiert.

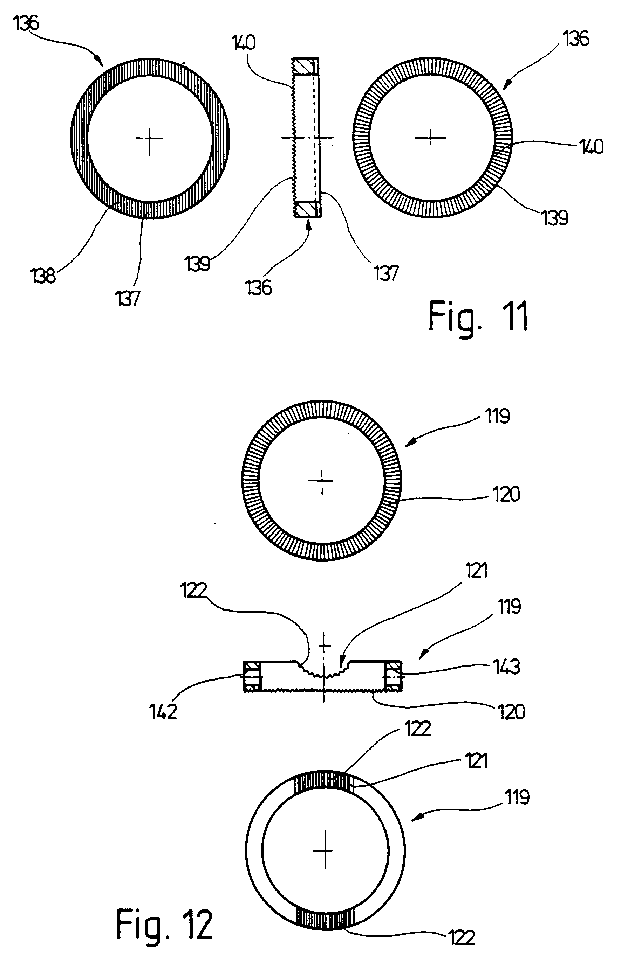

Figur 11 zeigt den über den Gabelkopf 109 schiebbaren Zwischenring 136, dessen proximale oder Unterseite 137 mit parallelen Kerben 138 und dessen distale oder Oberseite 139 mit einer radialen Stirnverzahnung (Hirth-Verzahnung) 140 versehen ist.

Der in Figur 12 dargestellte Überwurfring 119 besitzt im wesentlichen die Form des in den Figuren 3 und 4 dargestellten Überwurfringes 19 nach dem ersten Ausführungsbeispiel, mit der einzigen zusätzlichen Maßnahme, daß er umfangsseitig mit zwei diagonal gegenüberliegenden Bohrungen 142 und 143 versehen ist.

In zusammengebautem Zustand der Haltevorrichtung ist, wie Figur 8 zeigt, die Aufnahmevorrichtung 110 in die Knochenplatte 101 von der proximalen Seite her eingebracht, bevor die Knochenplatte 101 mit ihren Ankerstiften 106 in einen Knochen eingeschlagen und mit Hilfe von nicht dargestellten Befestigungsschrauben befestigt wird. Auf den Gabelkopf 109 der Aufnahmevorrichtung 110 wird zunächst der Zwischenring 136 aufgefädelt derart, daß die an der Unterseite 137 vorgesehenen Kerben 138 in die parallelen Kerben 108 der den Langloch-Durchbruch 107 umgebenden Stirn 132 der Knochenplatte 101 eingreifen können. Bevor diese parallelen Kerben 108 und 138 miteinander in Eingriff gebracht werden, wird die Aufnahmevorrichtung 110 mit dem Zwischenring 136 in Längsrichtung der Knochenplatte 101 in ihre gewünschte Position bewegt bzw. verschoben. Danach wird über den Gabelkopf 109, wie beim ersten Ausführungsbeispiel, der Überwurfring 119 geschoben, dessen radiale Stirnverzahnung 120 in die Stirnverzahnung 140 des Zwischenrings 136 nach einer Drehverstellung greifen kann. In die Bohrungen 142 und 143 des Überwurfrings 119 werden Stifte 144 und 145 eingebracht, die in die jeweilige axiale Nut 133, 134 am Außenumfang des Gabelkopfes 109 in Umfangsrichtung unbeweglich greifen und dadurch eine drehfeste Verbindung zwischen Überwurfring 119 und Aufnahmevorrichtung 110 herstellen. Auf diese Weise kann nach einem entsprechenden Verdrehen der Aufnahmevorrichtung 110 in die richtige Position gegenüber einem aufzunehmenden Korrekturstab der Gabelkopf 109 über den Überwurfring 119 am Zwischenring 136 drehfest fixiert werden, wenn die nicht dargestellte Fixiereinrichtung nach Einlegen eines ebenfalls nicht dargestellten Korrekturstabes aufgebracht und festgezogen ist.

Mit Hilfe der Konstruktion der zweiten Ausführungsform der knochenchirurgischen Haltevorrichtung ist es also möglich, den Gabelkopf 109 nicht nur gegenüber der senkrechten Achse zu verdrehen, sondern auch in Längsrichtung der Knochenplatte 101 zu verschieben, so daß ein Korrekturstab auch über drei oder mehr Haltevorrichtungen verlegt werden kann, ohne daß zuvor die jeweiligen Knochenplatten 101 in genauer Flucht zueinander angebracht sein müssen.

## Patentansprüche

1. Knochenchirurgische Haltevorrichtung für einen Korrekturstab (13), mit einer am Knochen befestigbaren Knochenplatte (1, 101), die ein Lager (17, 117) für eine Aufnahmevorrichtung (10, 110) für den Korrekturstab (13) aufweist, und mit einer den Korrekturstab (13) in der Aufnahmevorrichtung (10, 110) haltenden Fixiereinrichtung (27, 127), **dadurch gekennzeichnet**, daß die Aufnahmevorrichtung (10, 110) über die proximale Seite (28, 128) in das Lager (17, 117) einschiebbar ist und dabei die Knochenplatte (1, 101) durchgreift und auf der distalen Seite (29, 129) zur Aufnahme des Korrekturstabes (13, 113) überragt und die Knochenplatte (1, 101) mit eingeschobener Aufnahmevorrichtung (10, 110) und unabhängig von der Drehstellung der Aufnahmevorrichtung (10, 110) am Knochen fixierbar ist.

2. Knochenchirurgische Haltevorrichtung nach Anspruch 1, dadurch gekennzeichnet, daß die Aufnahmevorrichtung (10, 110) drehbar im Lager (17, 117) angeordnet ist.

3. Knochenchirurgische Haltevorrichtung nach Anspruch 1 oder 2, dadurch gekennzeichnet, daß die Knochenplatte (1) eine kreisrunde Durchgangsöffnung (7) aufweist, in der die Aufnahmevorrichtung (10) radial unbeweglich gehalten ist.

4. Knochenchirurgische Haltevorrichtung nach Anspruch 1 oder 2, dadurch gekennzeichnet, daß die Aufnahmevorrichtung (110) im Lager (117) in einer Richtung querschiebbar ist.

5. Knochenchirurgische Haltevorrichtung nach Anspruch 4, dadurch gekennzeichnet, daß die Knochenplatte (101) eine langlochartige Durchgangsöffnung (107) aufweist, in der die Aufnahmevorrichtung (110) querbeweglich gehalten ist, und daß das Lager (117) entsprechend der Durchgangsöffnung ebenfalls langlochartig ausgebildet ist.

6. Knochenchirurgische Haltevorrichtung nach mindestens einem der vorhergehenden Ansprüche, dadurch gekennzeichnet, daß die Aufnahmevorrichtung (10, 110) auf der proximalen Seite (28, 128) der Knochenplatte (1, 101) bündig mit dieser abschließt oder versenkt angeordnet ist.

7. Knochenchirurgische Haltevorrichtung nach mindestens einem der vorhergehenden Ansprüche, dadurch gekennzeichnet, daß die Knochenplatte (1, 101) mehrere Lager (17, 117) für entsprechend mehrere Aufnahmevorrichtungen (10, 110) aufweist.

8. Knochenchirurgische Haltevorrichtung nach mindestens einem der vorhergehenden Ansprüche, dadurch gekennzeichnet, daß die Aufnahmevorrichtung (10, 110) als Gabelkopf (9, 109) ausgebildet ist und an ihrem in der Knochenplatte (1, 101) zu verankernden Ende einen radial vorspringenden Bund (16, 116) aufweist, der das Lager (17, 117) der Knochenplatte (1, 101) zumindest teilweise hintergreift.

9. Knochenchirurgische Haltevorrichtung nach mindestens einem der vorhergehenden Ansprüche, dadurch gekennzeichnet, daß ein Überwurfring (19, 119) vorgesehen ist, der die Aufnahmevorrichtung (10, 110) aufnimmt.

10. Knochenchirurgische Haltevorrichtung nach Anspruch 9, dadurch gekennzeichnet, daß der Überwurfring (19) auf der distalen Seite (29) der Knochenplatte (1) aufliegt, und daß die an der Knochenplatte (1) anliegende Fläche des Überwurfringes (19) und die distale Seite (29) der Knochenplatte (1) eine das Lager (17) umgebende radiale Stirnverzahnung, Hirth-Verzahnung, (8, 20) aufweisen.

11. Knochenchirurgische Haltevorrichtung nach Anspruch 9 oder 7, dadurch gekennzeichnet, daß die distale Seite des Überwurfringes (19, 119) eine Diagonalnut (21, 121) zur Aufnahme des Korrekturstabes (13, 113) aufweist.

12. Knochenchirurgische Haltevorrichtung nach Anspruch 11, dadurch gekennzeichnet, daß die Diagonalnut (21, 121) insbesondere mit hinterschneidungsfreien Längskerben (22, 122) versehen ist, in die der mit zu den Längskerben (22, 122) entsprechende Längsnuten (30, 130) versehene Korrekturstab (13, 113) einsetzbar ist.

13. Knochenchirurgische Haltevorrichtung nach mindestens einem der Ansprüche 1, 2 und 4 bis 12, dadurch gekennzeichnet, daß ein Zwischenring (136) vorgesehen ist, der die Aufnahmevorrichtung (110) aufnimmt.

14. Knochenchirurgische Haltevorrichtung nach Anspruch 13, dadurch gekennzeichnet, daß der Zwischenring (136) zwischen der distalen Seite (129) der Knochenplatte (101) und der proximalen Seite (128) des Überwurfringes (119) angeordnet ist.

15. Knochenchirurgische Haltevorrichtung nach Anspruch 13 oder 14, dadurch gekennzeichnet, daß die an der Knochenplatte (101) anliegende Fläche des Zwischenringes (136) und die distale Seite (129) der Knochenplatte (101) eine das langlochartige Lager (117) umgebende Stirnverzahnung (108) aufweisen, die jeweils durch quer zur Längserstreckung des Lagers (117) verlaufende parallele Kerben (138) gebildet ist.

16. Knochenchirurgische Vorrichtung nach mindestens einem der Ansprüche 13 bis 15, dadurch gekennzeichnet, daß die an dem Überwurfring (119) anliegende Fläche des Zwischenringes (136) und die proximale Seite des Überwurfringes (119) mit einer radialen Stirnverzahnung, Hirth-Verzahnung, (120, 140) versehen sind.

17. Knochenchirurgische Haltevorrichtung nach mindestens einem der Ansprüche 1, 2, 4-9, 12-15, dadurch gekennzeichnet, daß zwischen der Aufnahmevorrichtung (110) und dem Überwurfring (119) eine zwar drehfeste jedoch axial bewegliche Verbindung vorgesehen ist.

18. Knochenchirurgische Haltevorrichtung nach Anspruch 17, dadurch gekennzeichnet, daß die Aufnahmevorrichtung (110) an mindestens einer Umfangsstelle eine axiale Nut (133, 134) aufweist, in die ein eine Radialbohrung (142, 143) des Überwurfringes (119) durchdringender Stift (144, 145) o.dgl. eingreift.

19. Knochenchirurgische Haltevorrichtung nach mindestens einem der vorhergehenden Ansprüche, dadurch gekennzeichnet, daß die Fixiereinrichtung (27, 127) eine auf die als Gabelkopf (9, 109) ausgebildete Aufnahmevorrichtung (10, 110) aufschraubbare Kopfmutter (23, 123), insbesondere eine Hutmutter, aufweist.

20. Knochenchirurgische Haltevorrichtung nach Anspruch 19, dadurch gekennzeichnet, daß die Kopfmutter (23, 123) mit einer koaxial einschraubbaren Fixierschraube (25, 125), insbesondere mit einem Innensechskant und/oder einer Ringschneide (26, 126), versehen ist.

21. Knochenchirurgische Haltevorrichtung nach mindestens einem der vorhergehenden Ansprüche, dadurch gekennzeichnet, daß die Knochenplatte (1, 101) wenigstens zwei bezüglich des Lagers (17, 117) einander gegenüberliegende Befestigungsbohrungen (2, 3; 102, 103) zur Aufnahme von Knochenschrauben aufweist.

22. Knochenchirurgische Haltevorrichtung nach Anspruch 21, dadurch gekennzeichnet, daß die distale Befestigungsbohrung (3, 103) schräg angeordnet ist, so daß die Knochenschraube in Richtung auf die gegenüberliegende Knochenschraube konvergiert.

23. Knochenchirurgische Haltevorrichtung nach Anspruch 21 oder 22, dadurch gekennzeichnet, daß die Befestigungsbohrungen (2, 3; 102, 103) jeweils ein kalottenförmiges Lager (5, 105) für den Schraubenkopf der Knochenschraube aufweisen.

## Claims

1. An orthopedic surgical holding device for a correcting rod (13), having a bone plate (1, 101) which can be fastened on the bone having a bearing for a receiver device (10, 110) for the correcting rod (13) and a fixing device (27) maintaining the correcting rod (13) in the receiver device (10, 110), characterized in that the receiver device (10, 110) can be pushed into the bearing (17, 117) from the proximal side (28, 128) and in the course of this extends through the bone plate (1, 101) and extends beyond it on the distal side (29, 129) for receiving the correcting rod (13) and that the bone plate (1, 101) with inserted receiver device (10, 110) can be fastened to the bone independently of the turned position of the receiver device (10, 110).

2. An orthopedic surgical holding device in accordance with claim 1, characterized in that the receiver device (10, 110) is rotatably disposed in the bearing (17, 117).

3. An orthopedic surgical holding device in accordance with claim 1, characterized in that the bone plate (1) has a circular through opening (7), in which the receiver device (10) is maintained radially immovable.

4. An orthopedic surgical holding device in accordance with claim 1 or 2, characterized in that the receiver device (110) can be pushed crosswise in one direction in the bearing (117).

5. An orthopedic surgical holding device in accordance with claim 4, characterized in that the bone plate (101) has a through opening (107) in the shape of an elongated hole, in which the receiver device (110) is maintained in a crosswise movable manner, and that the bearing (117) is also embodied in the shape of an elongated hole to correspond with the through opening.

6. An orthopedic surgical holding device in accordance with at least one of the preceding claims, characterized in that the receiver device (10, 110) terminates flush with the bone plate (1, 101) on the proximal side (28, 128) or is disposed recessed.

7. An orthopedic surgical holding device in accordance with at least one of the preceding claims, characterized in that the bone plate (1, 101) has a plurality of bearings (17, 117) for a corresponding plurality of receiver devices (10, 110).

8. An orthopedic surgical holding device in accordance with at least one of the preceding claims, characterized in that the receiver device (10, 110) is embodied as a fork head (9, 109) and has a radially projecting collar (16, 116) on its end to be anchored in the bone plate (1, 101) which grips the bearing (17, 117) of the bone plate (1, 101) at least partially from behind.

9. An orthopedic surgical holding device in accordance with at least one of the preceding claims, characterized in that a coupling ring (19, 119) is provided which receives the receiver device (10, 110).

10. An orthopedic surgical holding device in accordance with claim 9, characterized in that the coupling ring (19) is disposed resting on the distal side (29) of the bone plate (1), and the surface of the coupling ring (19) resting against the bone plate (1) and the distal side (29) of the bone plate (1) are provided with a radial front serration, Hirth serration, (8, 20) enclosing the bearing (17).

11. An orthopedic surgical holding device in accordance with claim 9 or 10, characterized in that the distal side of the coupling ring (19, 119) has a diagonal groove (21, 121) to receive the correcting rod (13).

12. An orthopedic surgical holding device in accordance with claim 11, characterized in that the diagonal groove (21, 121) is provided in particular with longitudinal notches (22, 122) which are free of undercuts, into which the correcting rod (13), provided with longitudinal grooves (30) corresponding to the longitudinal notches (22, 122), can be inserted.

13. An orthopedic surgical holding device in accordance with at least one of claims 1, 2 and 4 to 12, characterized in that an intermediate ring (136) is provided which receives the receiver device (110).

14. An orthopedic surgical holding device in accordance with claim 13, characterized in that the intermediate ring is disposed between the distal side (129) of the bone plate (101) and the proximal side (128) of the coupling ring (119).

15. An orthopedic surgical holding device in accordance with claim 13 or 14, characterized in that the surface of the intermediate ring (136) resting against the bone plate (101) and the distal side (129) of the bone plate (101) have a front serration (108) which encloses the bearing (117) which is in the shape of an elongated hole and which are respectively formed by parallel notches (138) extending crosswise to the long extension of the bearing (117).

16. An orthopedic surgical holding device in accordance with at least one of claims 13 to 15, characterized in that the surface of the intermediate ring (136) resting against the coupling ring (119) and the proximal side of the coupling ring (119) are provided with radial front serrations, Hirth serrations, (120, 140).

17. An orthopedic surgical holding device in accordance with at least one of claims 1, 2, 4 to 9, 12 to 15, characterized in that a connection fixed against relative rotation but axially moveable is provided between the receiver device (110) and the coupling ring (119).

18. An orthopedic surgical holding device in accordance with claim 17, characterized in that the receiver device (110) has an axial groove (133, 134) at least on one circumferential place, which is engaged by a pin (144, 145) or the like which extends through a radial bore (142, 143) of the coupling ring (119).

19. An orthopedic surgical holding device in accordance with at least one of the preceding claims, characterized in that the fixing device (27) has a head nut (23) in particular a cap nut, which can be screwed on the receiver device (10, 110) embodied as a fork head (9, 109).

20. An orthopedic surgical holding device in accordance with claim 19, characterized in that the head nut (23), is provided with a coaxially screwed in fixing screw (25), in particular with a hexagon socket and/or a cup point (26).

21. An orthopedic surgical holding device in accordance with at least one of the preceding claims, characterized in that the bone plate (1, 101) has at least two fastening bores (2, 3; 102, 103), which are located opposite each other in respect to the bearing (17, 117), for receiving bone screws.

22. An orthopedic surgical holding device in accordance with claim 21, characterized in that the distal fastening bore (3, 103) is disposed obliquely so that the bone screw converges in the direction toward the oppositely located bone screw.

23. An orthopedic surgical holding device in accordance with claim 21 or 22, characterized in that the fastening bores (2, 3; 102, 103) respectively have a cup-shaped bearing (5, 105) for the screw head of the bone screw.

## Revendications

1. Dispositif de fixation pour la chirurgie osseuse pour une broche de maintien (13) avec une plaque pour os (1, 101) se fixant sur l'os et qui présente un support (17, 117) pour un dispositif de logement (10, 110) pour la broche de maintien (13) et avec un dispositif de fixation (27) maintenant la broche de maintien (13) dans le dispositif de logement (10, 110), caractérisé en ce que le dispositif de logement (10, 110) est enfichable dans le support (17, 117) par le côté proximal (28, 128) et ainsi pénètre dans la plaque pour os (1, 101), et fait saillie par le côté distal (29, 129) pour loger la broche de maintien (13), et que la plaque pour os (1, 101) avec dispositif de logement (10, 110) enfiché peut être fixé sur l'os indépendamment de la position de rotation du dispositif de logement (10, 110).

2. Dispositif de fixation pour la chirurgie osseuse selon la revendication 1, caractérisé en ce que le dispositif de logement (10, 110) est disposé pivotant dans le support (17, 117).

3. Dispositif de fixation pour la chirurgie osseuse selon l'une quelconque des revendications 1 ou 2, caractérisé en ce que la plaque pour os (1) présente un trou de passage circulaire (7) dans lequel le dispositif de logement (10) est maintenu radialement fixe.

4. Dispositif de fixation pour la chirurgie osseuse selon l'une quelconque des revendications 1 ou 2, caractérisé en ce que le dispositif de logement (110) peut coulisser transversalement dans un sens dans le support (117).

5. Dispositif de fixation pour la chirurgie osseuse selon la revendication 4, caractérisée en ce que la plaque pour os (101) présente un trou de passage (107) en forme de trou longitudinal dans lequel le dispositif de logement (110) est maintenu de manière à pouvoir se déplacer transversalement et que le support (117) est aussi conçu en forme de trou longitudinal de façon analogue au trou de passage.

6. Dispositif de fixation pour la chirurgie osseuse selon l'une quelconque au moins des revendications précédentes, caractérisé en ce que le dispositif de logement (10, 110), sur le côté proximal (28, 118) de la plaque pour os (1, 101) est disposé bord à bord avec celle-ci ou encastré.

7. Dispositif de fixation pour la chirurgie osseuse selon l'une quelconque au moins des revendications précédentes, caractérisé en ce que la plaque pour os (1, 101) présente plusieurs supports (17, 117) pour plusieurs dispositifs de logements (10, 110) correspondants.

8. Dispositif de fixation pour la chirurgie osseuse selon l'une quelconque au moins des revendications précédentes, caractérisé en ce que le dispositif de logement (10, 110) est conçu comme une tête de fourche (9, 109) et présente à son extrémité qui vient s'ancrer dans la plaque pour os (1, 101) un bord radial en saillie (16, 116) qui prend le support (17, 117) de la plaque pour os (1, 101) du moins partiellement par l'arrière.

9. Dispositif de fixation pour la chirurgie osseuse selon l'une quelconque au moins des revendications précédentes, caractérisé en ce qu'une bague de fixation (19, 119) est prévue, qui accueille le dispositif de logement (10, 110).

10. Dispositif de fixation pour la chirurgie osseuse selon la revendication 9, caractérisé en ce que la bague de fixation (19) repose sur le côté distal (29) de la plaque pour os (1) et que la surface de la bague de fixation (19) qui repose sur la plaque pour os (1) et le côté distal (29) de la plaque pour os (1) présentent un crantage de surface radial, crantage Hirth (8, 20), qui entoure le support (17).

11. Dispositif de fixation pour la chirurgie osseuse selon l'une quelconque des revendications 9 ou 7, caractérisé en ce que le côté distal de la bague de fixation (19, 119) présente une rainure diagonale (21, 121) pour le logement de la broche de maintien (13).

12. Dispositif de fixation de la chirurgie osseuse selon la revendication 11, caractérisé en ce que la rainure diagonale (21, 121) est munie en particulier d'encoches longitudinales sans contre-dépouille (22, 122) dans lesquelles la broche de maintien (13) munie de rainures longitudinales (30) correspondant aux encoches longitudinales (22, 122) peut être placée.

13. Dispositif de fixation pour la chirurgie osseuse selon l'une quelconque au moins des revendications 1, 2 et 4 à 12, caractérisé en ce qu'une bague intermédiaire (136) est prévue, qui accueille le dispositif de logement (110).

14. Dispositif de fixation pour la chirurgie osseuse selon la revendication 13, caractérisé en ce que la bague intermédiaire (136) est disposée entre le côté distal (129) de la plaque pour os (101) et le côté proximal (128) de la bague de fixation (119).

15. Dispositif de fixation pour la chirurgie osseuse selon l'une quelconque des revendications 13 ou 14, caractérisé en ce que la surface de la bague intermédiaire (136) reposant sur la plaque pour os (101) et le côté distal (129) de la plaque pour os (101) présentent un crantage de surface (108) entourant le support en forme de trou longitudinal (117), chaque crantage étant formé d'encoches parallèles (136) qui s'étendent transversalement par rapport à l'étendue longitudinale du support (117).

16. Dispositif de fixation pour la chirurgie osseuse selon l'une quelconque au moins des revendications 13 à 15, caractérisé en ce que la surface de la bague intermédiaire (136) qui repose sur la bague de fixation (119) et le côté proximal de la bague de fixation (119) sont munis d'un crantage de surface radial, crantage Hirth (120, 140).

17. Dispositif de fixation pour la chirurgie osseuse selon l'une au moins des revendications 1, 2, 4-9, 12-15, caractérisé en ce qu'entre le dispositif de logement (110) et la bague de fixation (119), une jonction fixe mais cependant axialement mobile est prévue.

18. Dispositif de fixation pour la chirurgie osseuse selon la revendication 17, caractérisé en ce que le dispositif de logement (110) présente au moins sur une zone périphérique, une rainure axiale (133, 134) dans laquelle s'engrène une goupille (144, 145) ou similaire qui traverse un trou radial (142, 143).

19. Dispositif de fixation pour la chirurgie osseuse selon l'une quelconque au moins des revendications précédentes, caractérisé en ce que le dispositif de fixation (27) présente un écrou à tête (23) qui peut se visser sur le dispositif de logement (10, 110) conçu comme une tête de fourche (9, 109), en particulier un écrou borgne.

20. Dispositif de fixation pour la chirurgie osseuse selon la revendication 19, caractérisé en ce que l'écrou à tête (23) est muni d'une vis de fixation (25) qui peut être vissée coaxialement, en particulier d'une vis à six pans creux et/ou une cuvette (26).

21. Dispositif de fixation pour la chirurgie osseuse selon l'une quelconque au moins des revendications précédentes, caractérisé en ce que la plaque pour os (1, 101), concernant le support (17, 117), présente au moins deux trous de fixation (2, 3; 102, 103) placés l'un en face de l'autre pour le logement des vis pour os.

22. Dispositif de fixation pour la chirurgie osseuse selon la revendication 21, caractérisé en ce que le trou de fixation distal (3, 103) est disposé en biais de façon que la vis pour os converge en direction de la vis pour os opposée.

23. Dispositif de fixation pour la chirurgie osseuse selon l'une quelconque des revendications 21 ou 22, caractérisé en ce que les trous de fixation (2, 3; 102, 103) présentent chacun un support en forme de calotte (5, 105) pour la tête de vis de la vis pour os.
